# EUROPÄISCHE PATENTSCHRIFT

(11) **EP 1 663 120 B1**
(45) Veröffentlichungstag und Bekanntmachung des Hinweises auf die Patenterteilung: **10.12.2008**
(21) Anmeldenummer: 04729880.7
(22) Anmeldetag: 28.04.2004
(51) Int. Cl.: A61K 8/35, A61Q 5/04

(54) **VERWENDUNG VON BENZOCHINONEN IN MITTELN ZUR OXIDATIVEN BEHANDLUNG VON HAAREN BEI DER DAUERHAFTEN HAARVERFORMUNG SOWIE VERFAHREN ZUR DAUERHAFTEN HAARVERFORMUNG**
USE OF BENZOQUINONES IN COMPOSITIONS FOR THE OXIDATIVE TREATMENT OF HAIR IN PERMANENT SHAPING AND METHOD FOR PERMANENT SHAPING
UTILISATION DE BENZOQUINONES DANS DES COMPOSITIONS DE TRAITEMENT CAPILLAIRE OXYDATIF DANS LE CADRE DE LA MISE EN FORME PERMANENTE ET PROCEDE POUR LA MISE EN FORME PERMANENTE DES CHEVEUX

(30) Priorität: 26.06.2003 DE 10328667
(43) Veröffentlichungstag der Anmeldung: 07.06.2006
(73) Patentinhaber: Wella Aktiengesellschaft, 64274 Darmstadt (DE)
(72) Erfinder: ARLT, Thilo, CH-8112 Otelfingen (CH); CLÊMENT, Pierre, Alain, CH-1723 Marly (CH); BITTERLI, Natalie, CH-3280 Murten (CH)
(86) Internationale Anmeldenummer: PCT/EP2004/004465
(87) Internationale Veröffentlichungsnummer: WO 2005/000259

(56) Entgegenhaltungen:
- DE-A- 4 335 624
- DE-A- 19 617 486
- US-A- 4 560 554
- US-A- 5 180 400
- US-A- 5 275 626

## Beschreibung

Die vorliegende Erfindung betrifft die Verwendung von Benzochinonen in Mitteln zur Durchführung der oxidativen Nachbehandlung bei der dauerhaften Haarverformung sowie ein Verfahren zur dauerhaften Haarverformung.

Bei der reduktiven Veränderung von Keratinfasern wird das Haar zunächst mit einem Verformungsmittel, welches eine Öffnung der Disulfidbindungen des Haarkeratins bewirkt, behandelt und sodann in die gewünschte Form gebracht. Als Verformungsmittel werden hierbei in der Regel keratinreduzierende Mercaptoverbindungen, wie zum Beispiel Salze oder Ester von Mercaptocarbonsäuren, oder Sulfite verwendet. Anschließend wird das Haar mit Wasser oder einem geeigneten Zwischenbehandlungsmittel gespült. Sodann werden die reduzierten Haarfasern mit einem Fixiermittel oxidativ nachbehandelt. Hierbei werden Disulfidbindungen innerhalb des Haarkeratins geschlossen, welche für die dauerhafte Haltbarkeit der Haarverformung, insbesondere beim Wellen oder Glätten, maßgebend sind.

Bei Fixiermitteln auf der Basis von Hydrogenperoxid, Peroxidsalzen (Perborate, persulfate) oder Bromaten wird ein Teil der Disulfid- und Thiolgruppen des Haarkeratins zu höheren Oxidationsstufen des Schwefels, insbesondere zu Cysteinsäure aufoxidiert. Hierdurch wird das Haarkeratin irreversibel geschädigt. Im Falle peroxidhaltiger Fixiermittel werden außerdem die Farbpigmente des Haares (Melanine) partiell zerstört, was insbesondere bei asiatischem Haar mit einer Aufhellung des Haares verbunden ist.

Die Verwendung von Derivaten des Benzochinons zur Geruchsverbesserung in Fixierungen ist in Kombination mit herkömmlichen Oxidationsmitteln (vorzugsweise Bromat) aus US-PS 4560554 (24.12.1985; Shiseido) bekannt. Durch die Verwendung von Hydrogenperoxid als Oxidationsmittel wird das Haar jedoch durch Überoxidation geschädigt und besonders dunkles Haar in unerwünschter Weise aufgehellt.

Es stellte sich daher die Aufgabe, die bei der oxidativen Haarbehandlung, bei der dauerhaften Haarverformung, auftretenden Nachteile bezüglich Bleichwirkung und Cysteinsäurebildung zu vermeiden, ohne hierdurch die Haarstruktur zu beeinträchtigen.

Es wurde gefunden, dass die gestellte Aufgabe in hervorragender Weise durch die Verwendung von Benzochinonen in einem Mittel zur oxidativen Haarbehandlung bei der Nachbehandlung (Fixierung) von zuvor reduktiv behandelten Haaren bei der dauerhaften Haarverformung gemäß Anspruch 1 gelöst wird.

Unter Benzochinonen sollen p-Benzochinone sowie o-Benzochinone und deren wasserlösliche Derivate und Salze verstanden werden.

Für die erfindungsgemäße Verwendung besonders geeignet sind p-Benzochinone der Formel (I) und o-Benzochinone der Formel (II).

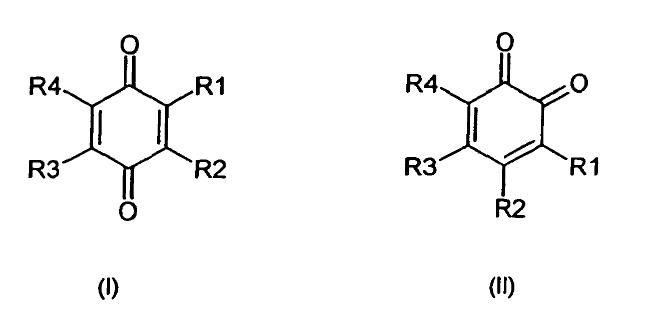

Es bedeuten R₁, R₂, R₃, R₄ unabhängig voneinander -H, -C₁ bis C₄-Alkyl, -Phenyl, -OH, -OR', -NH₂, -NHR'. -N(R')₂ , -N⁺(R')₃, -COOH, -COOR', -COR' oder -SO₃H, Halogen (F, Cl, Br, J) oder Pseudohalogen (SCN, CN) mit R' = C₁- bis C₄-Alkyl oder -Phenyl.

Besonders bevorzugt sind p-Benzochinone oder o-Benzochinone mit mindestens einem Substituenten verschieden von Wasserstoff, wobei dann R₁, R₂, R₃, R₄ die Bedeutung -H, -N⁺(R')₃, -COOH, -COOR', -COR', -SO₃H, Halogen (F, Cl, Br, J) oder Pseudohalogen (SCN, CN) mit R' = C₁- bis C₄-Alkyl oder -Phenyl haben.

Geeignet sind auch p-Benzochinone oder o-Benzochinone mit mindestens einem Substituenten verschieden von Wasserstoff, wobei R₁, R₂, R₃, R₄ die Bedeutung -H, -Alkyl, -Aryl, -OH, -OR', -NH₂, -NHR' oder-NR'₂ (R' = C₁- bis C₄-Alkyl oder-Phenyl) haben.

Ganz besonders bevorzugt sind wasserlösliche Benzochinon-Derivate mit ein bis vier Hydroxylgruppen, wie 2-Hydroxy-1,4-benzochinon, 3-Hydroxy-1,2-benzochinon, 2,5-Dihydroxy-1,4-benzochinon 3,4-Dihydroxy-1,2-benzochinon, Tetrahydroxy-1,4-benzochinon oder Tetrahydroxy-1,2-benzochinon.

Unter den Benzochinonen sind die o-Benzochinone, deren Derivate und deren Salze besonders bevorzugt.

Die Benzochinone sollen im gebrauchsfertigen Mittel in einer Menge von 0,1 bis 20 Gewichtsprozent, vorzugsweise 0,5 bis 10 Gewichtsprozent, besonders bevorzugt 1 bis 5 Gewichtsprozent, allein oder im Gemisch miteinander, verwendet werden.

Das Mittel zur oxidativen Behandlung von Haaren ist frei von haarschädigenden Oxidationsmitteln, insbesondere von Hydrogenperoxid, Peroxidsalzen oder Bromaten. Besonders bevorzugt sind die Benzochinone in dem Mittel zur oxidativen Behandlung von Haaren als einziges Oxidationsmittel enthalten.

Der pH-Wert des gebrauchsfertigen Mittels zur oxidativen Behandlung von Haaren liegt in einem Bereich von 1,5 bis 10, vorzugsweise 2,0 bis 8,0, besonders bevorzugt 2,5 bis 7,5. Die Einstellung des pH-Wertes erfolgt mit mindestens einem Mittel zur pH-Einstellung, wie Basen, Säuren und Puffersubstanzen, insbesondere mit Ammoniak, Ammonium- oder Alkalihydroxiden, Ammonium- oder Alkalicarbonat, Ammonium- oder Alkalihydrogencarbonat, Citronensäure und deren Salzen (Citratpuffer), Phosphorsäure und deren Salzen (Phosphatpuffer) oder Ascorbinsäure und deren Salzen, wobei Alkali vorzugsweise Natrium oder Kalium bedeutet und die Salze vorzugsweise die Chloride, Carbonate, Sulfate oder Phosphate sind.

Das gebrauchsfertige Mittel zur oxidativen Behandlung von Haaren kann sowohl in Form einer wässrigen oder wässrig-alkoholischen Lösung oder einer Emulsion als auch in verdickter Form auf wässriger Basis, insbesondere als Creme, Gel oder Paste vorliegen. Vorzugsweise werden die Benzochinone, deren Derivate oder Salze in wässriger oder in wäßrig-alkoholischer Lösung verwendet.

Neben den Benzochinonen, ihren Derivaten oder ihren Salzen oder deren Gemisch kann das gebrauchsfertige Mittel zur oxidativen Behandlung von Haaren noch Zusätze enthalten, wie sie in haarkosmetischen Zubereitungen üblich sind (vergleiche K. Schrader, Grundlagen und Rezepturen der Kosmetika, 2. Auflage, Hüthig-Verlag, Heidelberg, 1989). Hierzu gehören Quell- und Penetrationsstoffe, wie zum Beispiel Harnstoff, 2-Pyrrolidon, 1-Methyl-2-pyrrolidon und Dipropylenglykolmonomethylether, sowie Säuerungsmittel beispielsweise aromatische Sulfonsäuren, Salzsäure, Schwefelsäure, Phosphorsäure, Pyro- oder Polyphosphorsäuren, saure Salze starker Säuren, Ascorbinsäure, Oxalsäure, Malonsäure, Benzoesäure, Salicylsäure, Zitronensäure, Gerbsäuren.

Weiterhin können in dem Mittel zur oxidativen Behandlung von Haaren Netzmittel und Emulgatoren aus der Gruppe der anionischen, nichtionischen, kationischen und amphoteren oder zwitterionischen oberflächenaktiven Agenzien enthalten sein. Geeignete oberflächenaktive Agenzien sind insbesondere
a) anionische oberflächenaktive Agenzien, wie beispielsweise Alkali-, Erdalkali-, Ammonium- oder Alkanolaminsalze von Alkylsulfonaten, Alkylsulfaten und Alkylethersulfaten, wie zum Beispiel Natriumlaurylalkoholdiglykolethersulfat, Natrium- oder Triethanolaminsalze von Alkylsulfaten mit 12 bis 18, vorzugsweise 12 bis 14 Kohlenstoffatomen, die Natrium- oder Triethanolaminsalze von Lauryl- oder Tetradecylethersulfaten, das Dinatriumsalz des Sulfosuccinhalbesters von Alkanolamiden, Seifen und Polyethercarbonsäuren;
b) nichtionische oberflächenaktive Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl- und Stearylalkohol, allein oder im Gemisch, oxethylierte Lanolinalkohole, oxethyliertes Lanolin, oxethylierte Alkylphenole mit 8 bis 30 Kohlenstoffatomen im Alkylrest und 1 bis 10 Ethylenoxideinheiten im Molekül, Fettsäurealkanolamide sowie oxethylierte Sorbitanfettsäureester;
c) kationische oberflächenaktive Agenzien, wie beispielsweise Dilauryldimethylammoniumchlorid, Chloride oder Bromide von Alkyldimethylbenzylammoniumsalzen, Alkyltrimethylammoniumsalze, beispielsweise Cetyltrimethylammoniumchlorid oder bromid Tetradecyltrimethylammoniumchlorid oder - bromid, Alkyldimethylhydroxyethylammoniumchloride oder -bromide, Dialkyldimethylammoniumchloride oder -bromide, Alkylpyridiniumsalze, beispielsweise Lauryl- oder Cetylpyridiniumchlorid, Alkylamidoethyltrimethylammoniumethersulfate, Verbindungen mit kationischem Charakter wie Aminoxide, beispielsweise Alkyldimethylaminoxide oder Alkylaminoethyldimethylaminoxide und
d) amphotere oder zwitterionische oberflächenaktive Agenzien wie beispielsweise Carboxylderivate des Imidazols, N-Alkyl- und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureamidopropyldimethylaminoessigsäurebetain.

Selbstverstänblich kann das Mittel zur oxidativen Behandlung von Haaren alle für derartige Mittel üblichen Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, ferner Farbstoffe, Trübungsmittel, wie zum Beispiel Polyethylenglykolester, oder Alkohole, wie beispielsweise Ethanol, Propanol und Isopropanol, Lösungsvermittler, Puffersubstanzen, Parfümöle, haarkonditionierende oder haarpflegende Bestandteile, wie zum Beispiel Lanolinderivate, Cholesterin, Pantothensäure, Proteinderivate und -hydrolysate, Betain, Provitamine und Vitamine sowie Pflanzenextrakte enthalten.

Die Bestandteile der kosmetischen Zubereitung werden zur Herstellung der gebrauchsfertigen Mittel zur oxidativen Behandlung von Haaren in für diesen Zweck üblichen Mengen eingesetzt. Beispielsweise werden Netzmittel und Emulgatoren in Konzentrationen von 0,2 bis 30 Gewichtsprozent, Alkohole in einer Konzentration von 1 bis 80 Gew.%, haarkonditionierende oder haarpflegende Bestandteile in einer Konzentration von 0,1 bis 10 Gew.% und Verdickungsmittel in Konzentrationen von 0,1 bis 25 Gewichtsprozent bezogen auf das gebrauchsfertige Fixiermittel eingesetzt.

Das verwendete Mittel zur oxidativen Behandlung von Haaren ist ein Haardauerverformungsfixiermittel (nachfolgend "Fixiermittel" genannt).

Die Verwendung des gebrauchsfertigen Mittels zur oxidativen Behandlung von Haaren erfolgt in einem Temperaturbereich von 10°C bis 60°C, vorzugsweise 20°C bis 55°C, besonders bevorzugt 20°C bis 40°C. Die Einwirkungszeiten betragen von 1 bis 45 Minuten, vorzugsweise 2 bis 25 Minuten, besonders bevorzugt 3 bis 15 Minuten.

Besonders bevorzugt weist das verwendete Mittel zur oxidativen Behandlung von Haaren einen pH-Wert in einem Bereich von 1,5 bis 10, vorzugsweise 2,0 bis 8,0, besonders bevorzugt 2,5 bis 7,5 auf, enthält (A) 0,5 bis 10,0 Gew.% mindestens eines Benzochinons, dessen Derivats oder Salzes und (B) 0,01 bis 10 Gew.%. mindestens eines Mittels zur pH-Einstellung wie zum Beispiel Ammoniak, Ammonium- oder Alkalihydroxide, Ammonium- oder Alkalicarbonat, Ammonium- oder Alkalihydrogencarbonat, Citronensäure und deren Salze (Citratpuffer), Phosphorsäure und deren Salze (Phosphatpuffer) oder Ascorbinsäure und deren Salze, wobei Alkali vorzugsweise Natrium oder Kalium bedeutet und die Salze vorzugsweise die Chloride, Carbonate, Sulfate oder Phosphate sind.

Ein weiterer Gegenstand der vorliegenden Erfindung ist die Verwendung eines Mittel zur oxidativen Behandlung von Haaren bei der dauerhaften Haarverformung, dadurch gekennzeichnet, dass es frei von Hydrogenperoxid, Peroxidsalzen oder Bromaten ist und mindestens ein o-Benzochinon, ausgewählt aus o-Benzochinon, dessen Derivaten oder Salzen, enthält. Bevorzugt weist das o-Benzochinon die allgemeine Formel (II) auf. Ganz besonders bevorzugt ist das o-Benzochinonderivat ausgewählt aus 3-Hydroxy-1,2-benzochinon, 3,4-Dihydroxy-1,2-benzochinon und Tetrahydroxy-1,2-benzochinon.

Besonders bevorzugt weist das erfindungsgemäße Mittel zur oxidativen Behandlung von Haaren einen pH-Wert in einem Bereich von 1,5 bis 10, vorzugsweise 2,0 bis 8,0, besonders bevorzugt 2,5 bis 7,5 auf und enthält (A) 0,5 bis 10,0 Gew.% o-Benzochinon, ausgewählt aus o-Benzochinon, dessen Derivaten oder Salzen und (B) 0,01 bis 10 Gew.% mindestens eines Mittels zur pH-Einstellung, insbesondere Ammoniak, Ammonium- oder Alkalihydroxid, Ammonium- oder Alkalicarbonat, Ammonium- oder Alkalihydrogencarbonat, Citronensäure und deren Salze (Citratpuffer), Phosphorsäure und deren Salze (Phosphatpuffer) oder Ascorbinsäure und deren Salze, wobei Alkali vorzugsweise Natrium oder Kalium bedeutet und die Salze vorzugsweise die Chloride, Carbonate, Sulfate oder Phosphate sind.

Dieses erfindungsgemäß verwendete Mittel kann im übrigen die selben üblichen Zusatzstoffe in den selben Mengen enthalten, wie dies vorstehend für das bei der erfindungsgemäßen Verwendung beschriebene Mittel zur oxidativen Behandlung von Haaren angegeben ist.

Ein weiterer Gegenstand der vorliegenden Erfindung ist ein Verfahren zur dauerhaften Haarverformung, bei dem man das Haar bevor und/oder nachdem man es in die gewünschte Form bringt mit einem keratinreduzierenden Verformungsmittel behandelt, gegebenenfalls spült, sodann mit einem Fixiermittel oxidativ nachbehandelt, gegebenenfalls spült, anschließend zur Frisur legt und sodann trocknet, welches dadurch gekennzeichnet ist, dass für die oxidative Nachbehandlung das vorstehend beschriebene Mittel zur oxidativen Behandlung von Haaren als Fixiermittel verwendet wird. Die Spülung erfolgt vorzugsweise mit Wasser.

Bei einer besonderen Ausführungsform des erfindungsgemäßen Verfahrens wird das Haar zunächst mit dem keratinreduzierenden Verformungsmittel behandelt, das Verformungsmittel nach der Einwirkungszeit ausgespült, anschließend wird das Haar mit dem vorstehend beschriebenen Mittel zur oxidativen Behandlung von Haaren auf der Basis der Benzochinone, deren Derivate oder Salze als Oxidationsmittel behandelt (vorfixiert) und sodann mit einem Fixiermittel auf der Basis von Hydrogenperoxid oder Bromat nachbehandelt (nachfixiert). Besonders vorteilhaft weist das Fixiermittel für die Nachfixierung eine geringere Konzentration an Oxidationsmittel auf als für solche Mittel üblich; so beträgt vorzugsweise die Konzentration an Hydrogenperoxid nur 0,1 bis 1 Gew.% bzw. an Bromat nur 1 bis 5 Gew.%.

Bei dem erfindungsgemäßen Verfahren wird das Haar gewaschen, mit einem Handtuch frottiert, gegebenenfalls mit einem Teil des keratinreduzierenden Verformungsmittels vorgefeuchtet, in einzelne Strähnen aufgeteilt und auf Wickler gewickelt. Der Durchmesser der Wickler beträgt hierbei, je nach dem ob eine Dauerwellung oder eine Entkräuselung der Haare gewünscht wird, entweder etwa 5 bis 13 Millimeter oder etwa 15 bis 35 Millimeter. Auf das gewickelte Haar wird anschließend eine für die dauerhafte Haarverformung ausreichende Menge eines Verformungsmittels aufgetragen. Die für die dauerhafte Haarverformung erforderliche Gesamtmenge des Verformungsmittels beträgt im allgemeinen etwa 80 bis 120 Gramm.

Die bei dem erfindungsgemäßen Verfahren verwendbaren Verformungsmittel enthalten übliche keratinreduzierende Verbindungen, wie zum Beispiel bestimmte Mercaptoverbindungen, insbesondere Thioglykolsäure oder deren Salze, Thiomilchsäure oder deren Salze, Cystein, Cysteamin sowie Salze oder Ester von Mercaptocarbonsäuren. Diese Verformungsmittel enthalten die keratinreduzierenden Verbindungen in den für derartige Mittel üblichen Mengen, beispielsweise die Ammoniumsalze der Thioglykol- oder Thiomilchsäure in einer Menge von etwa 2 bis 12 Gewichtsprozent. Der pH-Wert dieser Verformungsmittel beträgt im allgemeinen etwa 7 bis 11, wobei die Einstellung des pH-Wertes vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmitteln werden vorzugsweise Ester von Mercaptocarbonsäuren, wie beispielsweise Monothioglykolsäureglykolester oder -glycerinester oder Sulfite, in einer Konzentration von etwa 2 bis 25 Gewichtsprozent verwendet.

Die Verformungsmittel können weiterhin alle für derartige Mittel üblichen Zusatzstoffe, beispielsweise Quell- und Penetrationsstoffe, Verdickungsmittel, Netzmittel und Emulgatoren, Alkohole, Lösungsvermittler, Stabilisatoren, Farbstoffe, Parfümöle sowie haarkonditionierende oder haarpflegende Bestandteile, enthalten. Die vorstehend genannten Zusatzstoffe werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in dem Verformungsmittel enthalten sein können.

Das bei dem erfindungsgemäßen Verfahren verwendete Verformungsmittel kann sowohl in Form einer wässrigen Lösung oder Emulsion als auch in verdickter Form auf wässriger Basis, insbesondere als Creme, Gel oder Paste, oder in Form eines Aerosolschaums vorliegen.

Nach einer für die dauerhafte Haarverformung ausreichenden Einwirkungszeit, welche je nach der Haarbeschaffenheit, dem pH-Wert und der Verformungswirksamkeit des Verformungsmittels sowie in Abhängigkeit von der Anwendungstemperatur etwa 5 bis 45 Minuten (5 bis 30 Minuten mit Wärmeeinwirkung; 20 bis 45 Minuten ohne Wärmeeinwirkung) beträgt, wird das Haar mit Wasser gespült und sodann mit etwa 50 Gramm bis 350 Gramm, vorzugsweise 80 bis 200 Gramm des vorstehend beschriebenen gebrauchsfertigen Fixiermittels oxidativ nachbehandelt.

Nach einer Einwirkungszeit des Fixiermittels von 1 bis 45 Minuten, vorzugsweise 3 bis 25 Minuten, besonders bevorzugt 5 bis 15 Minuten, werden die Wickler entfernt und das abgewickelte Haar, nur falls erforderlich, nochmals mit dem Fixiermittel oxidativ nachbehandelt. Sodann wird das Haar, vorzugsweise mit Wasser, gespült, zur Frisur gelegt und getrocknet.

Das so behandelte Haar besitzt eine gleichmäßige und haltbare Umformung. Im Unterschied zu Haaren, die mit Peroxid fixiert wurden und die insbesondere auf japanischen Haaren eine deutlich feststellbare Farbverschiebung in Richtung rot und gelb aufweisen (Aufhellungsgrad), liegen die Werte bei der erfindungsgemäß verwendeten Fixierung im Bereich unbehandelter Haarsträhnen. Zudem liegt der Cysteinsäuregehalt des so behandelten Haares deutlich unter dem von Haar, das mit einem Fixiermittel auf Basis von Hydrogenperoxid oder Bromat behandelt wurde.

### Beispiele

Die nachfolgenden Beispiele seien zur genauen Beschreibung des erfindungsgemäßen Verfahrens aufgeführt.

In diesen Beispielen wurde das Haar jeweils in folgender Weise reduktiv vorbehandelt:
Je 3 bis 4 unbehandelte und damit nicht vorgeschädigte Zählhaarsträhnen (bestehend entweder aus je 100 mittelbraunen, europäischen Haaren oder aus je 60 schwarzen, asiatischen Haaren jeweils mit einer Länge von genau 16,5 Zentimetern) wurden nass auf genormte Spiralwickler (Innendurchmesser: 3 Millimeter) aufgewickelt und nach dem Konditionieren im Exsikkator (Temperatur 20°C; Luftfeuchte: 99%) mit einer handelsüblichen Welllösung (Thioglykolsäuregehalt: 10 Gewichtsprozent; pH = 8,2) behandelt. Die Auftragemenge an Wellflüssigkeit wurde über das Verhältnis 1 : 1,2 errechnet (1 g Haar: 1,2 ml Wellflüssigkeit). Die Menge von 1,2 ml auf etwa 1 Gramm Haar entspricht einer Menge von 50 ml Wellösung pro Damenkopf mit einem Durchschnittsgewicht von ca. 30g Haar pro Kopf. Als Einwirkungszeit wurden 15 Minuten gewählt; die Einwirkungstemperatur des Reduktionsmittels betrug 50 Grad Celsius.

### Beispiel 1

Nach dem Ende der Einwirkungszeit wird die überschüssige Welllösung mit Wasser ausgespült und sodann die folgende Fixierlösung aufgetragen:

| | |
|---|---|
| 1,00 g | p-Benzochinon |
| 0,04 g | Phosphorsäure |
| ad 100,00 g | Wasser |

Der pH-Wert des Mittels liegt bei 2,5.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt einen guten Allgemeinzustand, ist nicht aufgehellt und zeigt einen erhöhten Glanz.

### Beispiel 2

Analog zu Beispiel 1 wird nach dem Ende der Einwirkungszeit die überschüssige Welllösung mit Wasser ausgespült und sodann folgende Fixierlösung aufgetragen:

| | |
|---|---|
| 1,00 g | o-Benzochinon |
| 0,02 g | Ammoniak, 25%-ige wässrige Lösung |
| ad 100,00 g | Wasser |

Der pH-Wert des Mittels liegt bei 6.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit, ist nicht aufgehellt und zeigt erhöhten Glanz.

### Beispiel 3

Analog zu Beispiel 1 wird nach dem Ende der Einwirkungszeit die überschüssige Welllösung mit Wasser ausgespült und sodann folgende Fixierlösung aufgetragen:

| | |
|---|---|
| 1,00 g | 2,5-Hydroxy-1,4-benzochinon |
| 2,14 g | Ammoniak, 25%-ige wässrige Lösung |
| ad 100,00 g | Wasser |

Der pH-Wert dieses Mittels liegt bei 6.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit, ist nicht aufgehellt und zeigt erhöhten Glanz.

### Beispiel 4

Analog zu Beispiel 1 wird nach dem Ende der Einwirkungszeit die überschüssige Welllösung mit Wasser ausgespült und sodann folgende Fixierlösung aufgetragen:

| | |
|---|---|
| 1,00 g | Tetrahydroxy-1,4-benzochinon |
| 0,38 g | Ammoniak, 25%-ige wässrige Lösung |
| ad 100,00 g | Wasser |

Der pH-Wert dieses Mittels liegt bei 6.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit, ist nicht aufgehellt und zeigt erhöhten Glanz.

## Patentansprüche

1. Verwendung von Benzochinonen als einziges Oxidationsmittel in Mitteln zur oxidativen Behandlung von Haaren, **dadurch gekennzeichnet, dass** das Mittel zur oxidativen Behandlung von Haaren ein Haardauerverformungsfixiermittel ist.

2. Verwendung nach Anspruch 1, **dadurch gekennzeichnet, dass** man als Benzochinon p-Benzochinon, o-Benzochinon oder deren wasserlösliche Derivate oder Salze verwendet.

3. Verwendung nach Anspruch 2, **dadurch gekennzeichnet, dass** man als wasserlösliches Benzochinonderivat Dihydroxy-benzochinon oder Tetrahydroxy-benzochinon verwendet.

4. Verwendung nach Anspruch 3, **dadurch gekennzeichnet, dass** man als Benzochinonderivat 2-Hydroxy-1,4-benzochinon, 3-Hydroxy-1,2-benzochinon, 2,5-Dihydroxy-1,4-benzochinon 3,4-Dihydroxy-1,2-benzochinon, Tetrahydroxy-1,4-benzochinon oder Tetrahydroxy-1,2-benzochinon verwendet.

5. Verwendung nach einem der Ansprüche 1 bis 4, **dadurch gekennzeichnet, dass** man das Benzochinon, dessen Salz oder Derivat in einer Menge von 1 bis 5 Gewichtsprozent verwendet.

6. Verwendung nach einem der Ansprüche 1 bis 5, **dadurch gekennzeichnet, dass** man das Benzochinon, dessen Derivat oder Salz bei einem pH-Wert von 2,5 bis 7,5 verwendet.

7. Verwendung nach einem der Ansprüche 1 bis 6, **dadurch gekennzeichnet, dass** das Mittel mindestens ein o-Benzochinon, ausgewählt aus o-Benzochinon, dessen Derivaten oder Salzen, enthält.

8. Verwendung nach Anspruch 7, **dadurch gekennzeichnet, dass** das Mittel einen pH-Wert in einem Bereich von 1,5 bis 10, aufweist und enthält:
(A) 0,5 bis 10,0 Gew.% mindestens eines o-Benzochinons, dessen Derivats oder dessen Salzes und
(B) 0,01 bis 10 Gew.% mindestens eines Mittels zur pH-Einstellung.

9. Verwendung nach Anspruch 8, **dadurch gekennzeichnet, dass** das Mittel zur pH-Einstellung ausgewählt ist aus Ammoniak, Ammonium- oder Alkalihydroxid, Ammonium- oder Alkalicarbonat, Ammonium- oder Alkalihydrogencarbonat, Citronensäure und deren Salzen, Phosphorsäure und deren Salzen oder Ascorbinsäure und deren Salzen, wobei Alkali Natrium oder Kalium bedeutet und die Salze die Chloride, Carbonate, Sulfate oder Phosphate sind.

10. Verfahren zur dauerhaften Haarverformung, bei dem man das Haar bevor und/oder nachdem man es in die gewünschte Form bringt, mit einem keratinreduzierenden Verformungsmittel behandelt, danach spült, sodann mit einem Fixiermittel oxidativ nachbehandelt und erneut spült, **dadurch gekennzeichnet, dass** man als Fixiermittel ein Mittel nach einem der Ansprüche 1 bis 9 verwendet.

## Claims

1. Use of benzoquinones as sole oxidizing agent in agents for the oxidative treatment of hair, **characterized in that** the agent for the oxidative treatment of hair is a hair permanent shaping neutralizer.

2. Use according to Claim 1, **characterized in that** the benzoquinone used is p-benzoquinone, o-benzoquinone or water-soluble derivatives or salts thereof.

3. Use according to Claim 2, **characterized in that** the water-soluble benzoquinone derivative used is dihydroxybenzoquinone or tetrahydroxybenzoquinone.

4. Use according to Claim 3, **characterized in that** the benzoquinone derivative used is 2-hydroxy-1,4-benzoquinone, 3-hydroxy-1,2-benzoquinone, 2,5-dihydroxy-1,4-benzoquinone, 3,4-dihydroxy-1,2-benzoquinone, tetrahydroxy-1,4-benzoquinone or tetrahydroxy-1,2-benzoquinone.

5. Use according to one of Claims 1 to 4, **characterized in that** the benzoquinone, its salt or derivative is used in an amount of from 1 to 5 per cent by weight.

6. Use according to one of Claims 1 to 5, **characterized in that** the benzoquinone, its derivative or salt is used at a pH of from 2.5 to 7.5.

7. Use according to one of Claims 1 to 6, **characterized in that** the agent comprises at least one o-benzoquinone, selected from o-benzoquinone, its derivatives or salts.

8. Use according to Claim 7, **characterized in that** the agent has a pH in a range from 1.5 to 10 and comprises:
(A) 0.5 to 10.0% by weight of at least one o-benzoquinone, its derivative or its salt and
(B) 0.01 to 10% by weight of at least one agent for adjusting the pH.

9. Use according to Claim 8, **characterized in that** the agent for adjusting the pH is selected from ammonia, ammonium or alkali metal hydroxide, ammonium or alkali metal carbonate, ammonium or alkali metal hydrogencarbonate, citric acid and salts thereof, phosphoric acid and salts thereof or ascorbic acid and salts thereof, where alkali metal means sodium or potassium and the salts are the chlorides, carbonates, sulphates or phosphates.

10. Method for the permanent shaping of hair, in which the hair, before and/or after it is brought into the desired shape, is treated with a keratin-reducing shaping composition, then rinsed, then oxidatively after-treated with a neutralizer and rinsed again, **characterized in that** the neutralizer used is an agent according to one of Claims 1 to 9.

## Revendications

1. Utilisation de benzoquinones en tant qu'oxydant unique dans des compositions destinées au traitement oxydatif des cheveux, **caractérisée en ce que** la composition destinée au traitement oxydatif des cheveux est un fixateur de mise en forme permanente des cheveux.

2. Utilisation selon la revendication 1, **caractérisée en ce que** l'on utilise, en tant que benzoquinone, la p-benzoquinone, la o-benzoquinone ou leurs dérivés ou sels hydrosolubles.

3. Utilisation selon la revendication 2, **caractérisée en ce que** l'on utilise, en tant que dérivé de benzoquinone hydrosoluble, la dihydroxybenzoquinone ou la tétrahydroxybenzoquinone.

4. Utilisation selon la revendication 3, **caractérisée en ce que** l'on utilise, en tant que dérivé de benzoquinone, la 2-hydroxy-1,4-benzoquinone, la 3-hydroxy-1,2-benzoquinone, la 2,5-dihydroxy-1,4-benzoquinone, la 3,4-dihydroxy-1,2-benzoquinone, la tétrahydroxy-1,4-benzoquinone ou la tétrahydroxy-1,2-benzoquinone.

5. Utilisation selon l'une quelconque des revendications 1 à 4, **caractérisée en ce que** l'on utilise la benzoquinone, son sel ou son dérivé en une quantité de 1 à 5 pour cent en poids.

6. Utilisation selon l'une quelconque des revendications 1 à 5, **caractérisée en ce que** l'on utilise la benzoquinone, son dérivé ou son sel à un pH de 2,5 à 7,5.

7. Utilisation selon l'une quelconque des revendications 1 à 6, **caractérisée en ce que** la composition comprend au moins une o-benzoquinone choisie parmi la o-benzoquinone, ses dérivés ou ses sels.

8. Utilisation selon la revendication 7, **caractérisée en ce que** la composition présente un pH dans une plage de 1,5 à 10 et comprend :
(A) de 0,5 à 10,0 % en poids d'au moins une o-benzoquinone, de son dérivé ou de son sel, et
(B) de 0,01 à 10 % en poids d'au moins un agent destiné à ajuster le pH.

9. Utilisation selon la revendication 8, **caractérisée en ce que** l'agent destiné à ajuster le pH est choisi parmi l'ammoniac, l'hydroxyde d'ammonium ou de métal alcalin, le carbonate d'ammonium ou de métal alcalin, l'hydrogénocarbonate d'ammonium ou de métal alcalin, l'acide citrique et ses sels, l'acide phosphorique et ses sels ou l'acide ascorbique et ses sels, où le métal alcalin représente le sodium ou le potassium et les sels sont les chlorures, les carbonates, les sulfates ou les phosphates.

10. Procédé de mise en forme permanente des cheveux, dans lequel les cheveux sont traités avec un agent de mise en forme réducteur de la kératine, avant et/ou après qu'ils ont été mis sous la forme souhaitée, puis rincés, ensuite soumis à un après-traitement oxydateur avec un fixateur et de nouveau rincés, **caractérisé en ce que** l'on utilise une composition selon l'une quelconque des revendications 1 à 9 en tant que fixateur.
